# EUROPÄISCHE PATENTANMELDUNG

(11) **EP 0 885 613 A1**
(43) Veröffentlichungstag der Anmeldung: **23.12.1998**
(21) Anmeldenummer: 97110168.8
(22) Anmeldetag: 21.06.1997
(51) Int. Cl.: A61K 38/42

(54) **Verwendung von modifizierten Hämoglobinen zur Behandlung von Anämien und Kombinationspräparate umfassend Erythropoietin und modifiziertes Hämoglobin**

(71) Anmelder: BOEHRINGER MANNHEIM GMBH, 68298 Mannheim (DE)
(72) Erfinder: Lehmann, Paul, Dr., Dipl.-Chem., 67549 Worms (DE)

(57) **Zusammenfassung**

Die Erfindung betrifft die Verwendung von modifizierten Hämoglobinen in Kombination mit Erythropoietin zur Behandlung von Anämien sowie ein Kombinationspräparat umfassend Erythropoietin und ein oder mehrere modifizierte Hämoglobine.

## Beschreibung

Die Erfindung betrifft die Verwendung von modifizierten Hämoglobinen in Kombination mit Erythropoietin (EPO) zur Behandlung von Anämien sowie ein Kombinationspräparat umfassend Erythropoietin und ein oder mehrere modifizierte Hämoglobine.

Bei der Diagnostik von Anämien spielt das Makromolekül Ferritin (Molekulargewicht mindestens 440 kD in Abhängigkeit vom Eisengehalt) eine bedeutende Rolle. Durch Bestimmung des Ferritins und der Transferrinsättigung ist eine Beurteilung des Füllstandes der Eisenspeicher möglich (M. Wick, W. Pingerra, P. Lehmann "Ferritin im Eisenstoffwechsel und Diagnostik der Anämien", Seiten 5 - 22, 38 - 50, 65 - 77, 94 - 97, 2. erweiterte Auflage 1994, Springer Verlag Wien, New York), wobei die Gesamtheit des als basisches Ferritin in den Depotorganen Leber. Milz und Knochenmark gespeicherten Eisens etwa 800 bis 1200 mg beträgt. Eine erniedrigte Ferritinkonzentration ist die entscheidende Kenngröße zum Erkennen von Eisenmangelzuständen und deren Unterscheidung von anderen Ursachen einer hvpochromen Anämie, wie z.B. chronische Entzündungen und Tumoren.

Es ist bekannt, transfusionsbedingte Anämien bei Hämodialysepatienten mit rekombinantem Erythropoietin (rhEPO) zu therapieren, wobei in der Regel parallel zur EPO-Therapie eine Eisensubstitution durchgeführt werden muß. Diese Eisensubstitution erfolgt durch intravenöse Applikation von Eisen(III)-Salzen, wobei auf dem deutschen Arzneimittelmarkt gegenwärtig zwei intravenös applizierbare Eisenpräparate zur Verfügung stehen. Dabei handelt es sich um die Medikamente "Ferrlecit" und "Ferrum Vites". "Ferrlecit" ist ein Eisen(III)-Gluconat-Komplex, während "Ferrum Vites" ein Eisen(III)-Oxid-Saccharat-Komplex ist.

Es hat sich allerdings gezeigt, daß im Falle manifester Anämien mit manifestem Eisenmangel und Eisenverwertungsstörungen (<30 mg/dl Ferritin) die Eisensubstitution mit den genannten Präparaten Nachteile hat da zur Behandlung manifester Anämien relativ große Mengen eines pharmakologisch unbedenklichen Eisen(III)-salzes infundiert werden müssen. Die Verwendung der o. g. Eisenpräparate birgt die Möglichkeit unerwarteter Kreislaufreaktionen bis hin zum Kollaps in sich. insbesondere wenn größere Mengen relativ schnell injiziert werden müssen.

In WO 96/15805 ist eine Hämoglobintherapie für die Hämodialyse beschrieben, nach der sehr niedrige Dosen Stroma-freies Hämoglobin über einen Zeitraum von 10 bis 45 Minuten zum Erreichen einer Hämostabilisierung und zur Vermeidung einer Blutdrucksenkung bei empfindlichen Patienten verabreicht werden. Diese beschriebene Therapie führt jedoch bei manifesten Eisenmangel-Anämien zu keinem Erfolg.

Es wurde nun gefunden, daß zur Behandlung von Anämien die Kombination modifizierter Hämoglobine mit rhEPO überraschend vorteilhaft ist. Die Verwendung der modifizierten Hämoglobine macht es möglich, relativ hohe Infusionsmengen von 50 - 100 mi Hämoglobin (ca. 100 - 200 mg Fe²⁺) zu verabreichen und hat darüber hinaus den Vorteil, daß das im Hämoglobin vorhandene Eisen(II) über Haptoglobin/Hämopexin und Leberpassage zu Eisen(III) oxidiert werden muß, und damit eine direkte Übertragung auf Transferrin umgangen wird.

Modifizierte Hämoglobine im Sinne der Erfindung sind cross-linked Hämoglobine oder cross-linked Hämoglobin-Polymerisate, wie z.B. Diacetylsalicylsäure (Diaspirin), cross-linked Hämoglobin (DCL-Hb) oder andere Blutersatzmittel auf der Basis modifizierter Hämoglobine, wie z.B. OPTRO™ (Produkt der Fa. Somagen).

Gegenstand der Erfindung sind auch Kombinationspräparate, die rhEPO und ein oder mehrere modifizierte Hämoglobine enthalten, wobei das rhEPO und das modifizierte Hämoglobin in getrennten Darreichungsformen oder in einer einheitlichen Darreichungsform vorliegen können.

Je nach klinischem Bild der Anämie werden 500 - 10.000 U rhEPO und 50 - 100 ml modifiziertes Hämoglobin eingesetzt. So wird bei manifesten Anämien ohne Eisenverteilungsstörungen erfindungsgemäß eine hohe Dosis Fe²⁺, ca. 80 - 100 ml (ca. 160 - 200 mg Fe²⁺), vorzugsweise 85-95 ml, in Form eines modifizierten Hämoglobins und eine geringere Dosis EPO zwischen 3.000 und 5.000 U verabreicht. Als modifiziertes Hämoglobin wird vorzugsweise DCL-Hb verwendet. Handelt es sich um Eisenverwertungsstörungen bei manifesten Anämien wird eine höhere EPO-Dosis von ca. 4.000 - 7.000 U EPO, vorzugsweise 6000 - 7000 U, insbesondere etwa 7.000 U EPO, und eine hohe Dosis Fe²⁺ von ca. 80 - 100 ml, vorzugsweise etwa 100 ml, verabreicht.

Erfindungsgemäß enthält das Kombinationspräparat 500 - 10.000 U rhEPO, insbesondere 3000 - 7000 U rhEPO, und 50 - 100 ml (100 - 200 mg Fe²⁺) eines oder mehrerer modifizierter Hämoglobine.

Zur Behandlung von Eisenverwertungsstörungen bei manifesten Anämien enthält das erfindungsgemäße Kombinationspräparat vorzugsweise 3.000 - 7.000 U rhEPO und 80 - 100 ml eines modifizierten Hämoglobins, vorzugsweise etwa 5.000 U rhEPO, und etwa 100 ml eines oder mehrerer modifizierter Hämoglobine. Zur Behandlung manifester Eisenmangelanämien enthält das Kombinationspräparat ebenfalls vorzugsweise 3.000 - 7.000 U rhEPO und 80 - 100 ml eines modifizierten Hämoglobins.

Die erfindungsgemäßen Konzentrationen an rhEPO und Hämoglobin-Fe²⁺ erlauben in ihrer Kombination eine optimale Anämiebehandlung, insbesondere die Behandlung manifester Anämien.

Bei der Anwendung der Kombinationspräparate ist es möglich, rhEPO und modifiziertes Hämoglobin in einer sogenannten fixen Kombination, d. h. in einer einzigen pharmazeutischen Formulierung zu verabreichen, in der beide Verbindungen enthalten sind. Dies kann z. B. eine Injektionslösung- bzw. Infusionslösung oder deren Lyophilisat sein, die beispielsweise in Ampullen abgefüllt sind. Diese Darreichungsform hat den Vorteil, daß das EPO bei der Herstellung und Lagerung der Darreichungsform durch das modifizierte Hämoglobin stabilisiert wird. Im Falle eines Lyophilisates wird nach dessen Auflösen das EPO durch das modifizierte Hämoglobin aktiviert. Die fixe Kombination der beiden Wirkstoffe in Form eines Lyophilisates hat den weiteren Vorteil der einfachen und sicheren Handhabung. Das Lyophilisat wird in der Ampulle durch Zugabe pharmazeutisch üblicher Injektionsmedien gelöst und intravenös appliziert.

Es ist auch möglich, EPO und das modifizierte Hämoglobin in Form von getrennten pharmazeutischen Formulierungen (freie Kombination) gleichzeitig oder aber auch nacheinander zu applizieren. Diese freie Kombination, die in einer Verpackungseinheit zur Verfügung gestellt werden kann, hat den Vorteil der größeren Flexibilität. So ermöglichen diese Darreichungsformen auch eine Applikation der modifizierten Hämoglobine 1-3 Tage vor der EPO-Applikation.

Die Herstellung der pharmazeutischen Darreichungsformen erfolgt nach üblichen, in der galenischen Technik bekannten Verfahren mit pharmazeutisch üblichen Hilfsstoffen.

Bei der Durchführung der Therapie sind die diagnostischen Parameter Ferritin und Transferrin-Sättigung zu kontrollieren. Der Ferritinwert ist im Normalbereich, wenn er 400 µg/l ± 50 % beträgt. Die Transferrinsättigung sollte 30 - 40 % betragen.

Nachfolgend soll die Erfindung anhand von Anwendungsbeispielen näher erläutert werden.

### Beispiel 1:

Patienten mit manifestem Eisenmangel (Ferritin < 12 ng/ml, Transferrinsättigung < 15 % und Hämoglobin < 12 g/dl) werden einmal pro Woche 5.000 U rhEPO und dreimal pro Woche 100 ml eines modifizierten Hämoglobinpräparates, vorzugsweise DCL-Hb, infundiert. Diese Behandlung wird weitere fünf Wochen wiederholt, bis die Werte für Ferritin, Transferrinsättigung und Hämoglobin bzw. Hämatokrit im Normalbereich liegen.

## Patentansprüche

1. Verwendung von einem oder mehreren modifizierten Hämoglobinen in Kombination mit EPO zur Anämiebehandlung.

2. Verwendung nach Anspruch 1, dadurch gekennzeichnet, daß die modifizierten Hämoglobine zur i.v.-Eisensubstitution bei EPO-Gabe eingesetzt werden.

3. Verwendung nach einem der Ansprüche 1 oder 2, dadurch gekennzeichnet, daß als modifizierte Hämoglobine DCL-Hb (Diaspirin cross-linked Hämoglobin) oder OPTRO™ eingesetzt werden.

4. Verwendung nach einem der Ansprüche 1 bis 3, dadurch gekennzeichnet, daß 500 - 10.000 U rhEPO, insbesondere 3.000 - 7.000 U rhEPO, und 50 - 100ml modifiziertes Hämoglobin verabreicht werden.

5. Verwendung nach einem der Ansprüche 1 bis 4, dadurch gekennzeichnet, daß zur Behandlung manifester Anämien ohne Eisenverteilungsstörungen 80 - 100 ml modifiziertes Hämoglobin und 3.000 - 5.000 U rhEPO verabreicht werden.

6. Verwendung nach Anspruch 1 bis 4, dadurch gekennzeichnet, daß zur Behandlung manifester Anämien mit Eisenverwertungsstörungen 4.000 - 7.000 U rhEPO und 80 - 100 ml modifiziertes Hämoglobin verabreicht werden.

7. Pharmazeutisches Kombinationspräparat umfassend rekombinantes humanes Erythropoietin und ein oder mehrere modifizierte Hämoglobine, wobei EPO und Hämoglobin in getrennten Darreichungsformen oder in einer einheitlichen Darreichungsform vorliegen können.

8. Kombinationspräparat nach Anspruch 7, dadurch gekennzeichnet, daß es 500 - 10.000 U rhEPO, insbesondere 2.000 - 7.000 U rhEPO, und 80 - 100 ml modifiziertes Hämoglobin enthält.

9. Kombinationspräparat nach Anspruch 7, dadurch gekennzeichnet, daß es 500 - 10.000 U rhEPO, insbesondere 6000 - 7000 U rhEPO, und 50 - 60 ml modifiziertes Hämoglobin enthält.
